(19) Europäisches Patentamt

European Patent Office

Office européen des brevets

(11) **EP 1 295 594 A1**

(12) **DEMANDE DE BREVET EUROPEEN**

(43) Date de publication:
   **26.03.2003 Bulletin 2003/13**

(51) Int Cl.⁷: **A61K 7/50**

(21) Numéro de dépôt: **02291970.8**

(22) Date de dépôt: **06.08.2002**

(84) Etats contractants désignés:
   **AT BE BG CH CY CZ DE DK EE ES FI FR GB GR
   IE IT LI LU MC NL PT SE SK TR**
   Etats d'extension désignés:
   **AL LT LV MK RO SI**

(30) Priorité: **20.09.2001 FR 0112151**

(71) Demandeur: **L'OREAL**
   **75008 Paris (FR)**

(72) Inventeurs:
   • **Guiramand, Carole**
     **78350 Jouy en Josas (FR)**
   • **Hurel, Valérie**
     **91190 Gif/s/Yvette (FR)**

(74) Mandataire: **Rasson, Catherine**
   **L'OREAL-DPI**
   **6 rue Bertrand Sincholle**
   **92585 Clichy Cedex (FR)**

(54) **Crème cosmétique moussante**

(57)  La présente demande concerne une composition moussante pour application topique, contenant au moins une cire et un système tensioactif tel qu'au moins une phase paracristalline hexagonale directe ou cubique apparaisse quand la température augmente au-delà de 30°C et que cette phase paracristalline reste présente jusqu'à au moins 45°C.

Le système tensioactif permettant d'obtenir l'apparition d'une telle phase paracristalline comprend de préférence au moins un tensioactif hydrosoluble et au moins un tensioactif insoluble dans l'eau. Il comporte de préférence au moins un savon hydrosoluble.

Ces compositions se présentent sous forme de crèmes et elles présentent une bonne stabilité et de bonnes qualités de mousse. Elles peuvent être utilisées notamment dans les domaines cosmétique ou dermatologique, comme produits de nettoyage ou de démaquillage de la peau, du cuir chevelu et/ou des cheveux.

**Description**

La présente invention a trait à une composition moussante rinçable constituant une crème pour application topique, contenant une cire et un système tensioactif particulier, et présentant une bonne stabilité physique jusqu'à au moins 45°C, ainsi qu'à son utilisation dans les domaines cosmétique ou dermatologique, notamment comme produits de nettoyage ou de démaquillage de la peau, du cuir chevelu et/ou des cheveux.

Le nettoyage de la peau est très important pour le soin du visage. Il doit être le plus performant possible car les résidus gras tels que l'excès de sébum, les restes des produits cosmétiques utilisés quotidiennement et les produits de maquillage notamment les produits « waterproof » résistants à l'eau, s'accumulent dans les replis cutanés et peuvent obstruer les pores de la peau et entraîner l'apparition de boutons.

On connaît plusieurs grands types de produits de nettoyage de la peau : les lotions et gels aqueux détergents moussants, les huiles et gels anhydres nettoyants rinçables et les crèmes moussantes.

Les huiles et gels anhydres rinçables ont une action nettoyante grâce aux huiles contenues dans ces formulations. Ces huiles permettent la solubilisation des résidus gras et la dispersion des pigments de maquillage. Ces produits sont efficaces et bien tolérés. Ils présentent l'inconvénient d'être lourds, de ne pas mousser et de ne pas conférer de sensation de fraîcheur à l'application, ce qui est pénalisant d'un point de vue cosmétique.

Par ailleurs, les lotions et gels aqueux détergents moussants ont une action nettoyante grâce aux tensioactifs qui mettent en suspension les résidus gras et les pigments des produits de maquillage. Ils sont efficaces et agréables à utiliser du fait qu'ils moussent et qu'ils sont facilement éliminés. Toutefois, les lotions sont généralement assez fluides, ce qui rend leur manipulation parfois délicate, et il est difficile d'épaissir les gels tout en gardant de bonnes propriétés moussantes.

Pour obtenir de bonnes performances moussantes tout en ayant une composition épaisse, on a cherché à préparer des crèmes moussantes. Toutefois, les crèmes moussantes présentent l'inconvénient d'être souvent instables à chaud, et difficiles à épaissir sans perdre stabilité et performances moussantes.

On entend ici par « crèmes moussantes » des compositions opaques, visqueuses, constituées généralement d'un milieu aqueux contenant un mélange de tensioactifs tels que les sels d'acides gras (savons) ou les tensioactifs synthétiques anioniques, non ioniques, amphotères, et d'autres additifs tels que par exemple des polymères, des polyols, des charges.

Ces crèmes destinées en particulier au nettoyage de la peau développent de la mousse quand elles sont mélangées avec de l'eau. Elles peuvent être utilisées de deux façons :

- la première utilisation consiste à étaler la crème dans les mains, à l'appliquer sur le visage ou sur le corps puis à la masser en présence d'eau pour développer la mousse directement sur le visage ou le corps.
- l'autre utilisation possible de ce type de produit consiste à développer la mousse dans les paumes des mains avant d'être appliquée sur le visage ou le corps.

Dans les deux cas, la mousse est ensuite rincée.

La plupart des crèmes moussantes actuellement disponibles dans le commerce sont instables au-dessus de 40°C. Cela signifie que, si elles sont entreposées quelques jours à cette température, elles présentent une démixtion macroscopique, aboutissant à une séparation en au moins deux phases. Les crèmes ainsi demixées à une température nettement supérieure à la température ambiante se révèlent hétérogènes après retour à la température ambiante et donc inutilisables du fait de la dégradation de la texture et des propriétés moussantes. On entend ici par température ambiante une température tempérée, c'est-à-dire d'environ 20 à 25°C.

Or, il est indispensable que ce type de produit soit stable sur une large plage de températures. En effet, au cours de sa vie, le produit peut être exposé à des températures allant de —20°C à +45°C minimum selon les conditions de climat, de stockage et/ou de transport. Par exemple, il faut qu'une crème transportée dans une voiture qui risque de rester longtemps sous le soleil, c'est-à-dire à une température atteignant facilement 50°C conserve sa stabilité. Il faut aussi que ces crèmes moussantes puissent être utilisées dans les pays chauds sans que leur transport et leur conservation ne posent de problème.

Il est bien connu qu'il est possible d'empêcher cette séparation de phases d'une crème moussante en augmentant, par addition de polymères ou de charges, la consistance du produit soumis à des températures de +40° à +45°C. Toutefois, dans ce cas, le produit devient très rigide à température ambiante tempérée et ne correspond plus aux propriétés recherchées pour l'application sur la peau, et notamment il devient difficile de le mélanger à l'eau et de le faire mousser.

Par ailleurs, la réalisation de crèmes plus visqueuses est délicate en terme de procédé (homogénéisation par turbine difficile, risque d'introduction d'air...) et pose le problème d'une dégradation de la qualité de mélange à l'eau lors de l'utilisation et le problème de démarrage en mousse qui est difficile.

Il subsiste donc le besoin d'une crème moussante, stable jusqu'à au moins 45°C, dont l'aspect de crème soit maintenu à température ambiante même après passage à une température plus élevée, ayant une viscosité suffisante pour constituer une crème tout en gardant une bonne stabilité et les propriétés requises pour un bon nettoyage, notamment de bonnes qualités de mélange à l'eau et de formation de mousse.

La demanderesse a découvert de manière surprenante qu'on pouvait atteindre le but de l'invention et obtenir une composition moussante se présentant sous forme d'une crème et ayant une bonne stabilité, même à des températures de +40 - +45°C, en incorporant une cire et en utilisant un système tensioactif tel qu'au moins une phase paracristalline de type hexagonale directe ou cubique apparaisse quand ladite composition est chauffée à une température supérieure à 30°C et que cette phase paracristalline reste présente jusqu'à au moins 45°C.

L'association de cire et du système tensioactif particulier dans la composition de l'invention permet d'obtenir une crème qui est à la fois très stable et épaisse et onctueuse, et qui donne une mousse ayant de bonnes qualités cosmétiques, de bonnes performances de mousse et un bon étalement sur la peau.

Pour obtenir la stabilité requise, on préfère que la phase paracristalline formée (ou cristal liquide) soit du type phase hexagonale directe. Il n'est pas nécessaire que cette phase paracristalline soit présente à température ambiante mais elle doit impérativement apparaître au delà d'une température comprise entre 30°C et 45°C.

Les crèmes moussantes, qui ne présentent pas une organisation de phases, telle que citée ci-dessus, n'ont généralement pas une stabilité satisfaisante à 45°C. A cette température, elles subissent une démixtion macroscopique entre au moins deux phases et elles sont ensuite inappropriées à l'utilisation recherchée quand elles sont de nouveau à la température ambiante.

Ainsi, la présente demande a pour objet une composition moussante pour application topique, contenant dans un milieu aqueux, au moins une cire et un système tensioactif tel qu'au moins une phase paracristalline de type hexagonale directe et/ou cubique apparaisse quand la température augmente au-delà de 30°C et que cette phase paracristalline reste présente jusqu'à au moins 45°C.

Selon un mode préféré de réalisation de l'invention, au moins une des étapes de préparation de la crème selon l'invention est réalisée dans un mélangeur-extrudeur à vis, notamment en vue d'introduire des quantités de cire plus importantes, et par exemple supérieures à 2 % en poids par rapport au poids total de la composition.

La composition de l'invention se présente sous forme d'une crème, c'est-à-dire un produit souple par opposition à un produit solide tel qu'un stick. Une crème a une viscosité à la température ambiante (environ 20-25°C) allant généralement d'environ 20 à 250 poises, soit 2 à 25 Pa.s, de préférence d'environ 50 à 240 poises, soit 5 à 24 Pa.s, et mieux 50 à 200 poises, soit 5 à 20 Pa.s, cette viscosité étant mesurée avec un Rhéomat 180.

La composition obtenue bien que contenant une cire donne une mousse qui s'étale de manière homogène et qui est de bonne qualité (bulles fines, bonne densité de mousse, douceur et facilité de rinçage).

La ou les phases paracristallines présentes au-delà de +30°C peuvent être de type hexagonale directe ou cubique, ou être un mélange de ces deux phases, ou un mélange d'une de ces phases ou de ces deux phases avec une phase de type lamellaire. La ou les phase(s) paracristalline(s) comporte(nt) de préférence au moins une phase hexagonale directe.

On donne dans la présente demande aux termes de phase lamellaire, phase hexagonale directe et phase cubique, les sens que leur donne habituellement l'homme du métier.

Ainsi, on entend par phase lamellaire (phase D selon EKWALL, voir Advances in Liquid Crystals, vol. 1, page 1-143, Acad. Press, 1975, Ed. G.H. Brown), une phase cristal liquide à symétrie plane, comprenant plusieurs bicouches d'amphiphile disposées en parallèle et séparées par un milieu liquide qui est généralement de l'eau.

On entend par phase hexagonale directe (phase F selon EKWALL, voir Advances in Liquid Crystals, vol. 1, page 1-143, Acad. Press, 1975, Ed. G.H. Brown), une phase cristal liquide correspondant à une arrangement hexagonal de cylindres parallèles constitués d'un amphiphile et séparés par un milieu liquide qui est généralement de l'eau. Dans une phase hexagonale directe, le milieu continu est aqueux.

On entend par phase cubique, une phase organisée d'une manière bi-polaire en domaines hydrophile et lipophile distincts, en contact étroit et formant un réseau tridimensionnel à symétrie cubique thermodynamiquement stable. Une telle organisation a été notamment décrite dans "La Recherche", Vol.23, pp.306-315, Mars 1992 et dans "Lipid Technology", Vol.2, n°2, pp.42-45, Avril 1990. Selon l'arrangement des domaines hydrophile et lipophile, la phase cubique est dite de type normal ou inverse. Le terme de phase cubique utilisé selon la présente invention regroupe bien entendu les différents types de phases cubiques.

Une description plus précise de ces phases peut être trouvée dans la Revue Française des Corps Gras, n°2, Février 1969, p 87 à 111, (Lachampt et Vila, « Textures des phases paracristallines »).

Pour identifier les phases constitutives de la crème, on peut utiliser différentes techniques, et notamment (1) les mesures par diffraction des rayons-X aux petits angles et aux grands angles et (2) l'observation par microscopie optique en lumière polarisée.

### Technique de diffraction des rayons-X

La technique de diffraction des rayons-X est connue comme étant l'une des plus pertinentes pour mettre en évidence les organisations de phases paracristallines, en particulier au sein d'un échantillon. Les mesures par diffraction des rayons X peuvent être effectuées à l'aide d'un générateur CGR Sigma 2060 équipé d'un tube Inel à anticathode Cu et d'une chambre à focalisation linéaire montée en transmission symétrique. Les échantillons sont introduits à température ambiante dans une cellule de mesure obturée par des fenêtres en Mylar ou en Capton et placée dans un porte-échantillon thermorégulé.

Les spectres de diffraction obtenus avec une longueur d'onde λ = 1,54 Angströms (Raie Kα du cuivre) sont enregistrés à l'aide d'un écran photostimulable au phosphore scanné par un module de balayage laser Molecular Dynamics PhosphorImager PSI. La distance détecteur / échantillon est réglée à 133 mm ce qui donne accès à des distances réticulaires comprises entre environ 3 et 110 Angströms. Les spectres sont enregistrés à différentes températures fixes.

Avec cette technique, les phases paracristallines sont caractérisées par la présence, aux petits angles de diffraction, d'une série de plusieurs raies fines dues à des réflexions de Bragg et correspondant à des distances : d1, d2.....dn avec des rapports de distance d1/d1, d1/d2, ...., d1/dn qui sont caractéristiques de chaque type de phase, comme indiqué par exemple dans « La structure des colloïdes d'association, I. Les phases liquides cristallines des systèmes amphiphile-eau », V. Luzzati, H. Mustachi, A. Skoulios et F. Husson, Acta Cryst. (1960), 13, 660-667 ou dans Biochimica et Biophysica Acta (1990), 1031, p. 1 à 69, de J.M. Seddon. Ainsi, pour une phase de structure lamellaire et en particulier pour la phase paracristalline de type lamellaire fluide généralement désignée par Lα et encore appelée phase lisse (« neat phase »), les rapports de distance sont égaux à : 1, 2, 3, 4, .....Pour la phase paracristalline de type hexagonal directe généralement désignée par H1 ou E et encore appelée phase médian (« middle phase »), les rapports de distance sont égaux à : 1, √3, 2, √7, ....Aux grands angles de diffraction, les phases paracristallines présentent une bande centrée sur une distance de l'ordre de 4,5 Angströms tandis que les phases cristallines conduisent à des raies fines.

### Observations par microscopie optique

Les observations par microscopie optique en lumière polarisée contribuent également à l'identification des phases paracristallines, en particulier lorsque le nombre de raies observées par diffraction des Rayons-X est insuffisant pour établir sans ambiguïté la nature des phases paracristallines en présence.

Les observations de microscopie optique en lumière polarisée sont effectuées par exemple à l'aide d'un microscope LABORLUX S (LEITZ) équipé d'un objectif de grossissement 10, d'un système de polariseurs croisés et d'une platine chauffante (METTLER FP80/FP82). L'échantillon est déposé entre lame et lamelle pour microscope, recouvert par une seconde lame et scellement de l'ensemble par l'intermédiaire d'un joint de Parafilm®. Les observations sont effectuées à diverses températures fixes ou en balayage de température à 2°C/min entre la température ambiante et environ 95°C.

Il est connu par exemple que les solutions micellaires isotropes sont non biréfringentes, que les phases paracristallines de type cubique sont également non biréfringentes et que les phases paracristallines de type lamellaire fluide, hexagonal directe ou inverse présentent en lumière polarisée diverses textures caractéristiques décrites par exemple dans « Textures des phases paracristallines rencontrées dans les diagrammes d'équilibre : agents de surface, lipides, eau », F. Lachampt et R.M. Vila, Revue Française des corps gras (1969), 2, 87-111 ou dans « the aqueous phase behavior of surfactants », Robert G. Laughlin Academic press, (1996)-p. 521-546.

### Caractéristiques de viscosité

Les compositions selon l'invention constituent des crèmes plus ou moins fluides, ayant, ainsi qu'indiqué ci-dessus, une viscosité à 25°C allant généralement d'environ 2 à 25 Pa.s, de préférence d'environ 5 à 24 Pa.s et mieux 5 à 20 Pa.s (viscosité mesurée avec un Rhéomat 180). Ces crèmes sont caractérisées par des valeurs des modules |G*|, G' et G", à la température de 25°C, allant de $10^2$ à $10^6$ Pa.

G*, G' et G" sont les paramètres viscoélastiques utilisés pour mesurer les propriétés physiques des fluides viscoélastiques comme expliqué dans « An introduction to rheology » de H.A. BARNES, J.F. HUTTON, K. WALTERS, pages 46 à 54, (Ed. Elsevier - 1989).

G* est le module complexe (complex modulus) et G' et G" sont les composantes de G* : G* = G' + iG". G' et G" sont respectivement le module de conservation (storage modulus) et le module de perte (loss modulus) et i est égal à $(-1)^{1/2}$. Les composantes G' et G" du module complexe sont obtenues à partir de la relation entre la contrainte sinusoïdale (oscillatory stress) et la déformation sinusoïdale (oscillatory strain).

Les mesures rhéologiques de |G*|, G' et G" sont effectuées généralement en utilisant un Rhéomètre Haake RS150, à la température de 25°C, avec des corps de mesure de géométrie cône — plan, le diamètre du cône et la dimension du plan étant de 20 mm et l'angle du cône de 1° et l'entrefer entre le cône et le plan étant de 0,05 mm.

Pour faire des mesures dynamiques de viscoélasticité (oscillatory measurements), on détermine d'abord le domaine viscoélastique linéaire, en soumettant l'échantillon à des contraintes sinusoïdales d'amplitudes croissantes et de fréquence constante. Les modules sont reportés en fonction de l'amplitude de la contrainte (amplitude of stress) ou de l'amplitude de la déformation (strain) afin de déterminer les limites du domaine viscoélastique linéaire. Après avoir identifié le domaine viscoélastique linéaire, des mesures dynamiques sont réalisées dans la zone viscoélastique linéaire, pour une valeur de déformation constante située dans le domaine viscoélastique linéaire et à fréquence variable. Le rhéomètre Haake RS150 peut couvrir une gamme de fréquences variant de 0,0001 to 100 Hz (soit 0,00063 to 628 rad/sec).

A partir des valeurs des amplitudes de la contrainte $\tau_0$, de la déformation $\gamma_0$, ainsi qu'à partir du déphasage $\delta$, on établit les relations suivantes :

$$|G^*| = \frac{\tau_0}{\gamma_0}$$

$$G' = |G^*| \cos \delta$$

$$G'' = |G^*| \sin \delta$$

$$G^* = G' + iG''$$

Système tensioactif

Le système tensioactif utilisé dans la composition de l'invention et permettant d'obtenir l'apparition d'une phase paracristalline lors d'un chauffage à au moins 30°C, comprend de préférence au moins un tensioactif hydrosoluble et au moins un tensioactif insoluble dans l'eau.

On entend par « tensioactif hydrosoluble » un tensioactif qui à une concentration de 20 g/l dans l'eau permutée à une température d'environ 25°C, donne une solution isotrope transparente.

On entend à l'inverse par « tensioactif insoluble dans l'eau » un tensioactif qui à une concentration de 20 g/l dans l'eau permutée à une température d'environ 25°C, donne une solution trouble indiquant la non solubilisation du tensioactif dans l'eau.

Tensioactifs hydrosolubles

On peut utiliser tout tensioactif soluble dans l'eau. Il s'agit de préférence de tensioactifs moussants, c'est-à-dire aptes à mousser en présence d'eau. Ce sont principalement des dérivés anioniques, non ioniques ou amphotères possédant des chaînes grasses suffisamment courtes pour que ces produits soient bien solubles à température ambiante dans le milieu solvant aqueux de la composition. On peut utiliser un tensioactif hydrosoluble ou un mélange de tels tensioactifs.

Comme tensioactifs hydrosolubles, on peut citer par exemple :

1. les tensioactifs anioniques

Selon un mode particulier de réalisation de l'invention, le système tensioactif utilisé comprend de préférence au moins un tensioactif anionique hydrosoluble, et plus particulièrement au moins un acide carboxylique ou un sel d'acide carboxylique hydrosoluble, sel qui est obtenu à partir de l'acide et d'une base. Les acides carboxyliques pouvant être utilisés sont des acides gras, comportant une chaîne alkyle linéaire ou ramifiée, saturée ou insaturée, ayant de 6 à 16 atomes de carbone et de préférence 10 à 14 atomes de carbone. Les sels de tels acides gras constituent des savons. Le fait qu'un savon soit hydrosoluble ou non dépend à la fois de la longueur de la chaîne alkyle et du contre-ion constituant le sel. Comme sels, on peut utiliser par exemple les sels alcalins, les sels alcalino-terreux, les sels d'ammonium, les sels d'aminoalcools et les sels d'aminoacides, et notamment les sels de sodium, de potassium, de magnésium, de triéthanolamine, de N-méthylglucamine, de lysine et d'arginine. Les bases (aussi appelées ci-après agents de saponification) susceptibles d'être utilisées pour obtenir ces sels peuvent être par exemple les bases minérales comme les hydroxydes de métaux alcalins (hydroxyde de sodium et potasse), les hydroxydes de métaux alcalino-terreux (de magnésium) ou l'hydroxyde d'ammonium ou encore les bases organiques comme la triéthanolamine, la N-méthylglucamine, la lysine et l'arginine. L'acide carboxylique peut être en particulier l'acide laurique ou l'acide myristique.

Comme savon hydrosoluble, on peut citer par exemple les sels de potassium des acides gras en C10 à C14, et notamment le sel de potassium de l'acide laurique, le sel de potassium de l'acide myristique et leurs mélanges.

Le savon est généralement introduit dans la composition sous forme de la base d'une part et de l'acide gras d'autre part, la formation du sel se faisant in situ. Ainsi, quand le savon hydrosoluble est constitué du sel de potassium de l'acide laurique et/ou du sel de potassium de l'acide myristique, la composition peut contenir alors de l'acide laurique et/ou de l'acide myristique avec une quantité suffisante de potasse pour former les sels de potassium de l'acide laurique et/ou de l'acide myristique.

Comme tensioactifs anioniques pouvant être utilisés dans la composition de l'invention comme tensioactif hydrosoluble, on peut citer par exemple, outre les acides carboxyliques et leurs sels cités ci-dessus, les acides carboxyliques éthoxylés et leurs sels ; les sarcosinates et acylsarcosinates et leurs sels tels que le lauroyl sarcosinate de sodium ; les taurates et méthyltaurates et leurs sels ; les iséthionates et acyl iséthionates, produits de réaction d'acides gras comportant de 10 à 22 atomes de carbone, avec l'acide isethionique, et leurs sels tels que l'iséthionate de sodium et le cocoyl-iséthionate de sodium ; les sulfosuccinates et leurs sels ; les alkylsulfates et alkyléthersulfates et leurs sels, notamment le laurylsulfate de sodium ou de triéthanolamine, et le lauryléther sulfate de sodium ou de potassium ; les monoalkyl- et dialkylesters d'acide phosphorique et leurs sels, tels que par exemple le mono- et dilauryl phosphate de sodium, le mono- et dilauryl phosphate de potassium, le mono- et dilauryl phosphate de triéthanolamine, le mono- et dimyristyl phosphate de sodium, le mono- et dimyristyl phosphate de potassium, le mono- et dimyristyl phosphate de diéthanolamine, le mono- et dimyristyl phosphate de triéthanolamine ; les alkane sulfonates et leurs sels ; les sels biliaires tels que les cholates, déoxycholates, taurocholates, taurodéoxycholates ; les lipoaminoacides et leurs sels tels que les acylglutamates mono- et di-sodiques ; les tensioactifs géminés, bipolaires, tels que décrits dans Surfactant Science series, vol.74 édité par Krister Homberg ; et leurs mélanges.

2. Tensioactifs amphotères et zwitterioniques

Comme tensioactifs amphotères ou zwitterioniques susceptibles d'être utilisés comme tensioactifs hydrosolubles, on peut citer par exemple les bétaïnes tels que la diméthylbétaïne, la coco-bétaïne et la coco-amidopropylbétaïne; les sulfobétaïnes tels que la coco-amidopropylhydroxysultaïne ; les alkylamphoacétates tels que cocoamphodiacétate ; et leurs mélanges.

3. Tensioactifs non ioniques

Comme tensioactifs non ioniques susceptibles d'être utilisés comme tensioactifs hydrosolubles, on peut citer par exemple les éthers de polyols, comportant des chaînes grasses (8 à 30 atomes de carbone), tels que les éthers gras de sorbitol ou de glycéryle oxyéthylénés ; les éthers et esters de polyglycérol ; les alcools gras polyoxyéthylénés qui sont des éthers formés d'unités d'oxyde d'éthylène et d'au moins une chaîne d'alcool gras ayant de 10 à 22 atomes de carbone, dont la solubilité dépend du nombre d'oxyde d'éthylène et de la longueur de la chaîne grasse ; par exemple, pour une chaîne grasse comportant 12 atomes de carbone, le nombre d'oxyde d'éthylène doit être supérieur à 7, et à titre d'exemple d'alcools gras polyoxyéthylénés, on peut citer les éthers d'alcool laurylique comportant plus de 7 groupes oxyéthylénés ; les alkyl polyglucosides dont le groupe alkyle comporte de 1 à 30 atomes de carbone, comme par exemple le décylglucoside, le laurylglucoside, le cétostéarylglucoside, le cocoyl-glucoside ; les alkyl glucopyranosides et alkylthioglucopyranosides ; les alkyl maltosides ; les alcoyl N méthylglucamides ; les esters de sorbitan polyoxyéthylénés qui comportent généralement de 1 à 100 unités d'éthylène glycol et de préférence de 2 à 40 unités d'oxyde d'éthylène (OE) ; les esters d'aminoalcools ; et leurs mélanges.

La quantité de tensioactif(s) hydrosoluble(s) dans la composition de l'invention peut aller par exemple de 10 à 50 % en poids (en matière active), de préférence de 15 à 35 % en poids par rapport au poids total de la composition. Selon un mode préféré de réalisation de l'invention, la composition de l'invention comprend au moins 15 % en poids et de préférence au moins 20 % en poids de tensioactif(s) hydrosoluble(s) par rapport au poids total de la composition.

Tensioactifs insolubles dans l'eau

Les tensioactifs insolubles dans l'eau apportent notamment la texture (consistance) de la composition finale. Par ailleurs, dans la plage de température comprise entre environ 25°C et 45°C, ces tensioactifs s'associent en partie avec les tensioactifs hydrosolubles pour contribuer à la formation de la phase paracristalline (de préférence hexagonale directe) qui est à l'origine de la stabilité du produit jusqu'à au moins 45°C.

Comme tensioactifs insolubles dans l'eau utilisés dans la composition selon l'invention, on peut citer notamment les acides carboxyliques et leurs sels insolubles dans l'eau, sels obtenus à partir de l'acide et d'une base. Ces sels constituent les savons insolubles dans l'eau. Les acides carboxyliques comportent une chaîne alkyle linéaire ou ramifiée, saturée ou insaturée, ayant de 6 à 30 atomes de carbone et de préférence 12 à 22 atomes de carbone. Pour les dérivés comportant une seule chaîne grasse saturée, la chaîne comprend avantageusement de 12 à 32 atomes de carbone, de préférence de 14 à 22 atomes de carbone et mieux de 16 à 20 atomes de carbone. Pour les dérivés comportant une chaîne grasse monoinsaturée ou polyinsaturée ou ramifiée, la chaîne comprend avantageusement de 16 à 34 atomes de carbone et de préférence de 18 à 24 atomes de carbone.

Comme acide carboxylique, on peut citer notamment l'acide palmitique et l'acide stéarique.

Comme sels, on peut utiliser les sels alcalins, les sels alcalino-terreux, les sels d'ammonium, les sels d'aminoalcools et les sels d'aminoacides, et notamment les sels de sodium, de potassium, de magnésium, de triéthanolamine, de N-méthylglucamine, de lysine et d'arginine. Les bases susceptibles d'être utilisées pour obtenir ces sels peuvent être par exemple les bases minérales comme les hydroxydes de métaux alcalins (hydroxyde de sodium et potasse), les hydroxydes de métaux alcalino-terreux (de magnésium) ou l'hydroxyde d'ammonium ou encore les bases organiques comme la triéthanolamine, la N-méthylglucamine, la lysine et l'arginine.

On peut citer par exemple comme savon insoluble, le sel de sodium des acides gras en C12 à C22 et le sel de potassium des acides gras en C16 à C22, et notamment le sel de potassium de l'acide palmitique et le sel de potassium de l'acide stéarique.

Le savon est généralement introduit dans la composition sous forme de la base d'une part et de l'acide gras d'autre part, la formation du sel se faisant in situ. Ainsi, quand le savon insoluble est constitué du sel de potassium de l'acide palmitique et/ou du sel de potassium de l'acide stéarique, la composition peut contenir alors de l'acide palmitique et/ou de l'acide stéarique avec une quantité suffisante de potasse (hydroxyde de potassium) pour former les sels de potassium de l'acide palmitique et/ou de l'acide stéarique.

Comme autres tensioactifs pouvant être utilisés dans la composition de l'invention comme tensioactif insoluble, on peut citer par exemple les tensioactifs insolubles non ioniques ou anioniques, comme les esters de glycéryle et d'acides gras comportant de 14 à 30 atomes de carbone, tels que le stéarate de glycéryle ; les dérivés de stérols et de phytostérols éventuellement oxyéthylénés ; les sels alcalins du cholestérol sulfate, et en particulier le sel de sodium ; les sels alcalins du cholestérol phosphate, et en particulier le sel de sodium ; les alcools gras polyoxyéthylénés comportant une chaîne oxyéthylénée ayant un petit nombre de groupes oxyéthylénés, et en particulier moins de 10 groupes oxyéthylénés ; les dialkylphosphates tels que les sels alcalins du dicétylphosphate, et en particulier les sels de sodium et de potassium ; les sels alcalins du dimyristylphosphate, et en particulier les sels de sodium et de potassium ; les lécithines ; les sphingomyélines ; les céramides ; et leurs mélanges.

La quantité de tensioactif(s) insoluble(s) dans l'eau dans la composition de l'invention peut aller par exemple de 5 à 50 % (en matière active), et de préférence de 5 à 35 % en poids par rapport au poids total de la composition.

Le système tensioactif (tensioactifs hydrosolubles et insolubles) est présent dans la composition de l'invention en une quantité, en matière active, pouvant aller par exemple de 20 à 65 % en poids, et va de préférence de 30 à 65 % en poids et mieux de 40 à 60 % en poids par rapport au poids total de la composition.

De préférence, le système tensioactif comprend une quantité de savon(s) hydrosoluble(s) d'au moins 10 % en poids par rapport au poids total de la composition, et une quantité totale de savons (hydrosolubles et insolubles) de préférence d'au moins 20 % en poids par rapport au poids total de la composition, et allant de préférence de 30 à 40 % en poids par rapport au poids total de la composition.

Comme cires utilisables dans la composition, on peut citer par exemple les cires d'origine animale telles que la cire d'abeille, la lanoline et ses dérivés ; les cires végétales telles que cire de candelilla, de carnauba ; les cires minérales telles que les cires microcristallines, la paraffine, la vaseline ; les cires synthétiques telles que le tétrastéarate de di-triméthylolpropane; et leurs mélanges. On utilise de préférence comme cires, la cire de carnauba, la cire de candelilla, la cire d'abeille, et leurs mélanges.

On peut de manière avantageuse faire varier les viscosités des crèmes obtenues (viscosité à 25°C allant de 2 à 25 Pa.s) et leurs propriétés d'étalement en choisissant des cires appropriées, car les cires indiquées ci-dessus présentent des points de fusion différents (de 50°C à 90°C) et des duretés différentes (de 1 MPa à 10 MPa (Mpa=méga pascals, dureté mesurée par le Texturomètre TA/TX2 de la société Rheo à 25°C).

La composition contient au moins 0.01% en poids d'une ou plusieurs cires par rapport au poids total de la composition. La quantité de cire peut aller par exemple de 0,01 à 20 % en poids, de préférence de 0,1 à 15 % en poids et mieux de 0,3 à 10 % en poids par rapport au poids total de la composition.

Le milieu aqueux des crèmes moussantes de l'invention étant destiné à une application topique est un milieu physiologiquement acceptable, c'est-à-dire compatible avec la peau, les muqueuses, le cuir chevelu, les yeux et/ou les cheveux. Il peut contenir, outre l'eau, un ou plusieurs solvants choisis parmi les alcools inférieurs comportant de 1 à 6 atomes de carbone, tels que l'éthanol ; les polyols tels que la glycérine ; les glycols comme le butylène glycol, l'isoprène glycol, le propylène glycol, les polyéthylène glycols tels que le PEG-8 ; le sorbitol ; les sucres tels que le glucose, le fructose, le maltose, le lactose, le sucrose ; et leurs mélanges. La quantité de solvant(s) dans la composition de l'invention peut aller de 0,5 à 30 % en poids et de préférence de 5 à 20 % en poids par rapport au poids total de la composition.

On peut incorporer dans la composition de l'invention, un ou plusieurs polymères, dans des concentrations préférentielles allant de 0,05 à 2 % en poids par rapport au poids total de la composition.

On peut citer comme exemples de polymères utilisables dans la composition de l'invention :

- les biopolymères polysaccharidiques comme la gomme de xanthane, la gomme de guar, les alginates, les celluloses modifiées ;
- les polymères synthétiques tels que les polyacryliques comme le CARBOPOL 980 commercialisé par la société GOODRICH, les copolymères acrylate/acrylonitrile tels que le HYPAN SS201 commercialisé par la société KINGSTON ;
- les composés inorganiques tels que les smectites, les hectorites modifiées ou non telles que les produits BENTONE commercialisés par la société RHEOX, les produits LAPONITE commercialisés par la société SOUTHERN CLAY PRODUCTS, le produit VEEGUM HS commercialisé par la société R.T. VANDERBILT ;
- leurs mélanges.

Les compositions de l'invention peuvent contenir aussi des adjuvants habituellement utilisés dans le domaine des nettoyants moussants comme les polymères cationiques du type polyquaternium, qui apportent douceur et onctuosité à la crème moussante. Ces polymères cationiques peuvent être choisis de préférence parmi les polymères suivants :

- Polyquaternium 5 tel que le produit MERQUAT 5 commercialisé par la société CALGON ;
- Polyquaternium 6 tel que le produit SALCARE SC 30 commercialisé par la société CIBA, et le produit MERQUAT 100 commercialisé par la société CALGON ;
- Polyquaternium 7 tel que les produits MERQUAT S, MERQUAT 2200 et MERQUAT 550 commercialisés par la société CALGON, et le produit SALCARE SC 10 commercialisé par la société CIBA ;
- Polyquaternium 10 tel que le produit Polymer JR400 commercialisé par la société AMERCHOL ;
- Polyquaternium 11 tel que les produits GAFQUAT 755, GAFQUAT 755N et GAFQUAT 734 commercialisés par la société ISP ;
- Polyquaternium 15 tel que le produit ROHAGIT KF 720 F commercialisé par la société ROHM ;
- Polyquaternium 16 tel que les produits LUVIQUAT FC905, LUVIQUAT FC370, LUVIQUAT HM552 et LUVIQUAT FC550 commercialisés par la société BASF ;
- Polyquaternium 22 tel que le produit MERQUAT 280 commercialisé par la société CALGON ;
- Polyquaternium 28 tel que le produit STYLEZE CC10 commercialisé par la société ISP ;
- Polyquaternium 39 tel que le produit MERQUAT PLUS 3330 commercialisé par la société CALGON ;
- Polyquaternium 44 tel que le produit LUVIQUAT CARE commercialisé par la société BASF ;
- Polyquaternium 46 tel que le produit LUVIQUAT HOLD commercialisé par la société BASF ;
- Polyquaternium 47 tel que le produit MERQUAT 2001 commercialisé par la société CALGON.

On peut aussi utiliser comme polymère cationique les guars cationiques telles que le produit JAGUAR commercialisé par la société RHODIA.

En outre, les compositions de l'invention peuvent contenir un ou plusieurs adjuvants habituellement utilisés dans le domaine cosmétique, choisis parmi les actifs cosmétiques ou dermatologiques, les parfums, les conservateurs, les séquestrants (EDTA), les pigments, les nacres, les charges minérales ou organiques telles que le talc, le kaolin, les poudres de silice ou de polyéthylène, les colorants solubles, les filtres solaires et leurs mélanges. Les quantités de ces différents adjuvants sont celles classiquement utilisées dans le domaine considéré, et par exemple de 0,01 à 20 % du poids total de la composition. Ces adjuvants ainsi que leurs concentrations doivent être tels qu'ils ne modifient pas la propriété recherchée pour la composition de l'invention.

Comme actifs, on peut citer tout particulièrement les actifs antisébohrréïques et antimicrobiens; permettant notamment de traiter les peaux grasses. Cet actif peut notamment être choisi parmi : les dérivés de β-lactame, les dérivés de quinolone, la ciprofloxacine, la norfloxacine, la tétracycline et ses sels, l'érythromycine et ses sels, l'amikacine et ses sels, le 2,4,4'-trichloro-2'-hydroxy diphényl éther (ou triclosan), le 3,4,4'-trichlorocarbanilide (ou triclocarban), le phénoxyéthanol, le phénoxypropanol, le phénoxyisopropanol, la doxycycline et ses sels, la capréomycine et ses sels, la chlorhexidine et ses sels, la chlortétracycline et ses sels, l'oxytétracycline et ses sels, la clindamycine et ses sels, l'éthambutol et ses sels, l'hexamidine iséthionate, le métronidazole et ses sels, la pentamycine et ses sels, la gentamicine et ses sels, la kanamycine et ses sels, la linéomycine et ses sels, la méthacycline et ses sels, la méthénamine et ses sels, la minocycline et ses sels, la néomycine et ses sels, la netilmicine et ses sels, la paromomycine et ses sels, la streptomycine et ses sels, la tobramycine et ses sels, le miconazole et ses sels, les sels d'amantadine, le para-chloro-méta-xylénol, la nystatine, le tolnaftate, l'acide salicylique et ses sels, l'acide n-octanoyl-5 salicylique et ses sels, le peroxyde de benzoyle, l'acide 3-hydroxy benzoïque, l'acide 4-hydroxy benzoïque, l'acide acétylsalicylique, l'acide 2-hydroxybutanoïque, l'acide 2-hydroxypentanoïque, l'acide 2-hydroxyhexanoïque, l'acide phytique, l'acide N-acétyl-L-cystéine, l'acide lipoïque, l'acide azélaïque, l'acide arachidonique, l'ibuprofène, le naproxène, l'hydrocortisone, l'acétaminophène, le résorcinol, le 2,4,4'-trichloro-2'-hydroxy diphényl éther, le 3,4,4'-trichlorocarbanilide, l'octopirox, le chlorhydrate de lidocaïne, le clotrimazole, l'octoxyglycérine, l'octanoylglycine, le caprylylglycol, l'acide 10-hydroxy-2-décanoïque, les sels de zinc tels que le gluconate de zinc, la niacinamide ou vitamine B3 (ou vitamine PP), et leurs mélanges.

Comme actifs, on peut utiliser aussi dans la composition de l'invention tout actif habituellement utilisé dans les domaines cosmétique et dermatologique, tels que par exemple les vitamines ou provitamines hydrosolubles ou liposolubles comme les vitamines A (rétinol), C (acide ascorbique), B3 ou PP (niacinamide), B5 (panthénol), E (tocophérol), K1, le bêta-carotène, et les dérivés de ces vitamines et notamment leurs esters ; les stéroïdes comme la DHEA et la 7α-hydroxy DHEA ; les hydratants comme la glycérine, l'acide hyaluronique, le pyrrolidone carboxylique acide (PCA) et ses sels, le pidolate de sodium, la sérine, le xylitol, le tréhalose, l'ectoïne, les céramides, l'urée ; les agents kératolytiques et anti-âge tels que les alpha-hydroxyacides comme l'acide glycolique, l'acide citrique, l'acide lactique, les béta-hydroxyacides comme l'acide salicylique et ses dérivés ; les enzymes et co-enzymes et notamment le coenzyme Q10 ; les filtres solaires ; les azurants optiques ; les actifs amincissants comme la caféine, la théophylline, la théobromine, les anti-inflammatoires tels que les acides 18 β glycyrrhétinique et ursolique, et leurs mélanges.

On peut utiliser un mélange de deux ou plusieurs de ces actifs. Le ou les actifs peuvent être par exemple présents en une concentration allant de 0,01 à 20 %, de préférence de 0,1 à 10 % et mieux de 0,5 à 5 % du poids total de la composition.

Les compositions selon l'invention peuvent constituer notamment des crèmes moussantes pour application topique, utilisées en particulier dans les domaines cosmétique ou dermatologique, comme produits de nettoyage ou de démaquillage de la peau (corps ou visage y compris yeux), du cuir chevelu et/ou des cheveux. Elles peuvent constituer plus particulièrement une composition de nettoyage de la peau.

Un autre objet de l'invention consiste en l'utilisation cosmétique de la composition telle que définie ci-dessus, comme produits de nettoyage et/ou de démaquillage de la peau, du cuir chevelu et/ou des cheveux.

Un autre objet de l'invention consiste en un procédé cosmétique de nettoyage de la peau, du cuir chevelu et/ou des cheveux, caractérisé par le fait qu'on applique la composition de l'invention, sur la peau, sur le cuir chevelu et/ou sur les cheveux, en présence d'eau, et qu'on élimine la mousse formée et les résidus de salissure par rinçage à l'eau.

La composition selon l'invention peut être préparée de manière avantageuse en utilisant, pour au moins une étape du procédé de préparation, un appareil de malaxage tel qu'un broyeur à cylindres comportant deux cylindres tournant en sens inverse entre lesquels passe la pâte, ou un mélangeur-extrudeur à vis. On utilise de préférence un mélangeur-extrudeur à vis. Ce procédé se révèle en particulier avantageux quand la composition contient un taux important de cire, et notamment plus de 2 % de cire.

Un autre objet de l'invention est donc un procédé de préparation d'une composition selon l'invention, caractérisé en ce que l'on effectue au moins une étape du procédé à l'aide d'un mélangeur-extrudeur à vis.

Selon un premier mode de réalisation de l'invention, le procédé de préparation comprend les étapes suivantes :

- (1) préparation d'un prémélange des cires et autres composés solides à température ambiante (environ 25°C), tels que notamment les acides gras destinés à constituer les savons avec l'agent de saponification, en chauffant ce prémélange à une température à laquelle il fond (environ à 80°C),
- (2) incorporation de la phase aqueuse préchauffée également à 80°C, dans le prémélange de l'étape (1) et mélange des deux phases ;
- (3) introduction du mélange obtenu dans la partie chauffée à 80°C d'un mélangeur-extrudeur à vis, ce mélangeur-extrudeur étant soumis à un gradient de température allant de 80°C à 10°C ;
- (4) malaxage du mélange dans le mélangeur-extrudeur, tout en le refroidissant jusqu'à 10°C (température du dernier élément de l'extrudeur) tandis qu'il est amené à la sortie du mélangeur-extrudeur ; et
- (5) incorporation d'un agent de saponification (base) dans l'émulsion, dans une partie du mélangeur-extrudeur où la température est proche de la température ambiante (environ 25°C).

Les éléments du mélangeur-extrudeur à vis utilisé sont, en allant du premier au sixième élément, portés respectivement aux températures suivantes : 80°C, 80°C, 80°C, 30°C, 10°C et 10°C.

L'utilisation du mélangeur-extrudeur permet ainsi d'incorporer des taux de cires beaucoup plus importants (2%, 5%, 10%) que les mélangeurs classiques de type rotor-stator et d'obtenir de façon reproductible une crème de qualité très constante, et de viscosité allant de 20 à 250 poises (2 à 25 Pa.s).

Les exemples qui suivent servent à illustrer l'invention sans toutefois présenter un caractère limitatif. Les quantités indiquées sont en % en poids sauf mention contraire.

Tableau I

| | Exemple 1 selon l'invention | Exemple 2 selon l'invention | Exemple 3 selon l'invention | Exemple 4 selon l'invention |
|---|---|---|---|---|
| acide laurique | 3 | 3 | 3 | 3 |
| acide myristique | 20 | 20 | 20 | 20 |
| acide palmitique | 3 | 3 | 3 | 3 |
| acide stéarique | 3 | 3 | 3 3 | |
| Stéarate de glycéryle (nom CTFA : glyceryl stearate SE) | 5 | 5 | 5 | 5 |
| cire de carnauba | **1** | / | / | / |
| cire de candelilla | / | / | **2** | **5** |
| cire d'abeille | / | **2** | / | / |
| coco-glucoside (50% en matière active) | 1 % en M.A. | / | / | / |
| glycérine | 7 | 7 | 7 | 7 |
| PEG 8 | 7 | 7 | 7 | 7 |
| conservateurs | 0,7 | 0,7 | 0.7 | 0,7 |
| séquestrant | 0,2 | 0,2 | 0,2 | 0,2 |
| Potasse | 7 | 7 | 7 | 7 |
| eau | QSP100 | QSP100 | QSP100 | QSP100 |
| Aspect | produit blanc à reflets nacrés jaunes | produit blanc nacré | produit blanc nacré | produit blanc nacré |
| pH à 24h | 9,2 | 9,2 | 9,3 | 9,3 |
| Stabilité 2 mois toutes températures températures | Conforme | Conforme | Conforme | Conforme |
| | Bords nets, pas de relarguage, pas d'évolution de couleur ni d'odeur | Bords nets, pas de relarguage, pas d'évolution de couleur ni d'odeur | Bords nets, pas de relarguage, pas d'évolution de couleur ni d'odeur | Bords nets, pas de relarguage, pas d'évolution de couleur ni d'odeur |
| Viscosité viscosité | 212 poises (21,2 Pa.s) | 236 poises (23,6 Pa.s) | 165 poises (16,5 Pa.s) | 236 poises (23,6 Pa.s) |

Les compositions de l'invention présentent l'avantage d'avoir une viscosité suffisante pour constituer une crème qui ne coule pas et qui peut être conditionnée dans des pots comme une crème de soin, tout en gardant une bonne stabilité et tout en ayant de bonnes performances moussantes, comme le montrent les tableaux 2 et 3 indiqués ci-dessous.

Caractéristiques de viscosité des compositions indiquées ci-dessus :

Tableau 2

| | oscillation | balayage | | |
|---|---|---|---|---|
| Type de fabrication | formule | $|G^*|$ à 1 Hz (Pa) | G' à 1 Hz (Pa) | G" à 1 Hz (Pa) |

Tableau 2   (suite)

|  | | oscillation | balayage | | |
|---|---|---|---|---|---|
| Mélangeur classique | Exemple 1 selon l'invention | 137700 | 127800 | 51220 | |
| Mélangeur-extrudeur | Exemple 1 selon l'invention | 48970 | 43680 | 22140 | |
| | Exemple 3 selon l'invention | 54980 | 48840 | 25260 | |
| | Exemple 4 selon l'invention | 113800 | 110400 | 27780 | |

Ces résultats montrent :

- que l'ajout de cire à différents taux dans les compositions moussantes provoque une augmentation de la viscosité (avec mélangeur classique et avec extrudeur).
- et que les fabrications avec l'extrudeur permettent d'introduire de fort taux de cires avec un comportement rhéologique acceptable.

Performances sensorielles : les qualités de mousse développées sont évaluées selon le protocole décrit ci-dessous.

Avant toute utilisation des produits, les mains sont lavées au savon de Marseille puis convenablement rincées et séchées. Puis le protocole suivi est le suivant :

1- mouiller les mains en les passant sous l'eau courante, les secouer trois fois pour les essorer,
2- placer 1 g de produit dans le creux de l'une des mains,
3- travailler le produit entre les deux paumes pendant 10 secondes,
4- ajouter 2 ml d'eau et travailler le produit à nouveau pendant 10 secondes,
5- rincer les mains sous l'eau ,
6- les essuyer.

Les critères sont évalués à chaque étape du protocole suivi, et ils sont notés sur une échelle de 0 à 10.

- étape 4 : évaluation de la qualité de mousse

  - *Le volume de mousse:* la note attribuée est d'autant plus élevée que le volume est grand.
  - *La taille des bulles composant la mousse :* la note attribuée est d'autant plus élevée que les bulles sont grosses.
  - *La densité :* consistance, tenue de la mousse ; la note attribuée est d'autant plus élevée que la densité est grande.
  - *La douceur de la mousse :* la noté attribuée est d'autant plus élevée que la mousse est douce.

- étape 5 : évaluation pendant le rinçage

  - *Le rinçage :* la note attribuée est d'autant plus faible que la présence d'un film glissant difficile à éliminer est grande.

Les résultats sensoriels pour chacun des critères sont présentés dans le tableau 3 suivant :

Tableau 3

| Critères | Exemple selon l'invention |
|---|---|
| Volume de mousse | 5.9 ± 1.0 |
| Taille de bulle | 3 ± 1.4 |
| Densité de mousse | 7.6 ± 0.8 |
| Douceur de mousse | 8.7 ± 0.8 |
| Rinçage | 8.4 ± 1.1 |

Ces résultats montrent que la composition selon l'invention tout en ayant une viscosité et une stabilité satisfaisantes, a également de bonnes qualités de mousse, et notamment qu'elle permet d'obtenir une mousse fine (petite taille de bulles), de bonne densité, très douce et facile à rincer.

**Revendications**

1. Composition moussante pour application topique, contenant dans un milieu aqueux, au moins une cire et un système tensioactif tel qu'au moins une phase paracristalline de type hexagonale directe et/ou cubique apparaisse quand la température augmente au-delà de 30°C et que cette phase paracristalline reste présente jusqu'à au moins 45°C.

2. Composition selon la revendication 1, **caractérisée en ce que** la phase paracristalline comprend au moins une phase hexagonale directe.

3. Composition selon la revendication 1 ou 2, **caractérisée en ce qu'**elle a un module complexe |G*|, un module de conservation G' et un module de perte G" ayant des valeurs allant, à la température de 25°C, de $10^2$ à $10^6$ Pa.

4. Composition selon l'une quelconque des revendications précédentes, **caractérisée en ce que** le système tensioactif comprend au moins un tensioactif hydrosoluble et au moins un tensioactif insoluble dans l'eau.

5. Composition selon la revendication précédente, **caractérisée en ce que** le système tensioactif comprend au moins un tensioactif anionique hydrosoluble.

6. Composition selon la revendication précédente, **caractérisée en ce que** le tensioactif anionique hydrosoluble est choisi parmi les acides carboxyliques et leurs sels, les acides carboxyliques éthoxylés et leurs sels, les sarcosinates et acylsarcosinates et leurs sels, les taurates et méthyltaurates et leurs sels, les iséthionates et acyl iséthionates et leurs sels, les sulfosuccinates et leurs sels, les alkylsulfates et alkyléthersulfates et leurs sels, les monoalkyl- et dialkylesters d'acide phosphorique et leurs sels, les alkane sulfonates et leurs sels, les sels biliaires, les lipoaminoacides et leurs sels, les tensioactifs géminés, et leurs mélanges.

7. Composition selon la revendication 4, **caractérisée en ce que** le tensioactif hydrosoluble est un tensioactif amphotère ou zwitterionique choisi parmi les bétaïnes, les sulfobétaïnes, les alkylamphoacétates, et leurs mélanges.

8. Composition selon la revendication 4, **caractérisée en ce que** le tensioactif hydrosoluble est un tensioactif non ionique choisi parmi les éthers de polyols, les éthers et esters de polyglycérol, les alcools gras polyoxyéthylénés, les alkyl polyglucosides, les alkyl glucopyranosides et alkylthioglucopyranosides, les alkyl maltosides, les alcoyl N méthylglucamides, les esters de sorbitan polyoxyéthylénés, les esters d'aminoalcools, et leurs mélanges.

9. Composition selon la revendication 4, **caractérisée en ce que** le tensioactif insoluble dans l'eau est choisi parmi les acides carboxyliques et leurs sels ; les esters de glycéryle et d'acides gras ; les dérivés de stérols et de phytostérols éventuellement oxyéthylénés ; les sels alcalins du cholestérol sulfate ; les sels alcalins du cholestérol phosphate ; les alcools gras polyoxyéthylénés ; les dialkylphosphates ; les lécithines ; les sphingomyélines ; les céramides ; et leurs mélanges.

10. Composition selon l'une quelconque des revendications précédentes, **caractérisée en ce que** le système tensioactif est présent en une quantité, en matière active, allant de 20 à 65 % en poids par rapport au poids total de la composition.

11. Composition selon l'une quelconque des revendications 4 à 10, **caractérisée en ce qu'**elle comprend de 10 à 50 % en poids de tensioactif(s) hydrosoluble(s) par rapport au poids total de la composition.

12. Composition selon l'une quelconque des revendications 4 à 11, **caractérisée en ce qu'**elle comprend au moins 15 % en poids de tensioactif(s) hydrosoluble(s) par rapport au poids total de la composition.

13. Composition selon l'une quelconque des revendications précédentes, **caractérisée en ce que** le système tensioactif comprend au moins 10 % en poids de savon(s) hydrosoluble(s) par rapport au poids total de la composition.

14. Composition selon l'une quelconque des revendications 4 à 13, **caractérisée en ce qu'**elle comprend de 5 à 50 % en poids de tensioactif(s) insoluble(s) dans l'eau par rapport au poids total de la composition.

**15.** Composition selon l'une quelconque des revendications précédentes, **caractérisée en ce que** le système tensioactif comprend une quantité totale de savons d'au moins 20 % en poids par rapport au poids total de la composition.

**16.** Composition selon l'une quelconque des revendications précédentes, **caractérisée en ce que** la cire est choisie parmi les cires d'origine animale ; les cires végétales ; les cires minérales ; les cires synthétiques ; et leurs mélanges.

**17.** Composition selon l'une quelconque des revendications précédentes, **caractérisée en ce qu'**elle comprend au moins 0,01 % en poids de cire par rapport au poids total de la composition.

**18.** Composition selon l'une quelconque des revendications précédentes, **caractérisée en ce qu'**elle comprend de 0,01 à 20 % en poids de cire par rapport au poids total de la composition

**19.** Composition selon l'une quelconque des revendications précédentes, **caractérisée en ce qu'**elle comprend au moins un solvant choisi parmi les alcools inférieurs ; les polyols ; les sucres et leurs mélanges.

**20.** Composition selon l'une quelconque des revendications précédentes, **caractérisée en ce qu'**elle comprend au moins un polymère cationique.

**21.** Composition selon l'une quelconque des revendications précédentes, **caractérisée en ce qu'**elle comprend un ou plusieurs adjuvants choisis parmi les actifs cosmétiques, les parfums, les conservateurs, les séquestrants, les pigments, les nacres, les charges minérales ou organiques, les colorants solubles, les filtres solaires et leurs mélanges.

**22.** Composition selon la revendication précédente, **caractérisée en ce que** l'actif est choisi parmi les dérivés de β-lactame, les dérivés de quinolone, la ciprofloxacine, la norfloxacine, la tétracycline et ses sels, l'érythromycine et ses sels, l'amikacine et ses sels, le triclosan, le triclocarban, le phénoxyéthanol, le phénoxypropanol, le phénoxyisopropanol, la doxycycline et ses sels, la capréomycine et ses sels, la chlorhexidine et ses sels, la chlortétracycline et ses sels, l'oxytétracycline et ses sels, la clindamycine et ses sels, l'éthambutol et ses sels, l'hexamidine iséthionate, le métronidazole et ses sels, la pentamycine et ses sels, la gentamicine et ses sels, la kanamycine et ses sels, la linéomycine et ses sels, la méthacycline et ses sels, la méthénamine et ses sels, la minocycline et ses sels, la néomycine et ses sels, la netilmicine et ses sels, la paromomycine et ses sels, la streptomycine et ses sels, la tobramycine et ses sels, le miconazole et ses sels, les sels d'amantadine, le para-chloro-méta-xylénol, la nystatine, le tolnaftate, l'acide salicylique et ses sels, l'acide n-octanoyl-5 salicylique et ses sels, le peroxyde de benzoyle, l'acide 3-hydroxy benzoïque, l'acide 4-hydroxy benzoïque, l'acide acétylsalicylique, l'acide 2-hydroxybutanoïque, l'acide 2-hydroxypentanoïque, l'acide 2-hydroxyhexanoïque, l'acide phytique, l'acide N-acétyl-L-cystéine, l'acide lipoïque, l'acide azélaïque, l'acide arachidonique, l'ibuprofène, le naproxène, l'hydrocortisone, l'acétaminophène, le résorcinol, le 2,4,4'-trichloro-2'-hydroxy diphényl éther, le 3,4,4'-trichlorocarbanilide, l'octopirox, le chlorhydrate de lidocaïne, le clotrimazole, l'octoxyglycérine, l'octanoylglycine, le caprylylglycol, l'acide 10-hydroxy-2-décanoïque, les sels de zinc tels que le gluconate de zinc, la niacinamide, et leurs mélanges.

**23.** Composition selon l'une quelconque des revendications précédentes, **caractérisée en ce qu'**elle constitue une composition de nettoyage de la peau.

**24.** Utilisation cosmétique d'une composition selon l'une quelconque des revendications 1 à 22, comme produits de nettoyage et/ou de démaquillage de la peau, du cuir chevelu et/ou des cheveux.

**25.** Procédé cosmétique de nettoyage de la peau, du cuir chevelu et/ou des cheveux, **caractérisé en ce qu'**on applique la composition selon l'une quelconque des revendications 1 à 22, sur la peau, sur le cuir chevelu et/ou sur les cheveux, en présence d'eau, et qu'on élimine la mousse formée et les résidus de salissure par rinçage à l'eau.

**26.** Procédé de préparation d'une composition selon l'une quelconque des revendications 1 à 23, **caractérisé en ce que** l'on effectue au moins une étape du procédé à l'aide d'un mélangeur-extrudeur à vis.

**27.** Procédé selon la revendication 26, **caractérisé en ce qu'**il comprend les étapes suivantes :

- (1) préparation d'un prémélange des cires et autres composés solides à température ambiante, en chauffant ce prémélange à une température à laquelle il fond,
- (2) incorporation de la phase aqueuse préchauffée à 80°C, dans le prémélange de l'étape (1) et mélange des deux phases ;
- (3) introduction du mélange obtenu dans la partie chauffée à 80°C d'un mélangeur-extrudeur à vis, ce mélangeur-extrudeur étant soumis à un gradient de température allant de 80°C à 10°C ;
- (4) malaxage du mélange dans le mélangeur-extrudeur, tout en le refroidissant jusqu'à 10°C tandis qu'il est amené à la sortie du mélangeur-extrudeur ; et
- (5) incorporation d'un agent de saponification dans l'émulsion dans une partie du mélangeur-extrudeur où la température est proche de la température ambiante.

## RAPPORT DE RECHERCHE EUROPEENNE

**Office européen des brevets**

**Numéro de la demande**

EP 02 29 1970

### DOCUMENTS CONSIDERES COMME PERTINENTS

| Catégorie | Citation du document avec indication, en cas de besoin, des parties pertinentes | Revendication concernée | CLASSEMENT DE LA DEMANDE (Int.Cl.7) |
|---|---|---|---|
| A | EP 0 968 704 A (L'ORÉAL) 5 janvier 2000 (2000-01-05) * revendications 1-25 * | 1-27 | A61K7/50 |
| A | US 5 952 285 A (J. HAWKINS) 14 septembre 1999 (1999-09-14) * colonne 1, ligne 22 - colonne 21, ligne 67; revendications 1,2 * | 1 | |
| P,X | EP 1 184 031 A (L'ORÉAL) 6 mars 2002 (2002-03-06) * revendications 1-24; exemples 1,2 * | 1-27 | |
| P,X | EP 1 166 747 A (L'ORÉAL) 2 janvier 2002 (2002-01-02) * revendications 1-20; exemples 1,2 * | 1-27 | |

**DOMAINES TECHNIQUES RECHERCHES (Int.Cl.7)**

A61K

Le présent rapport a été établi pour toutes les revendications

| Lieu de la recherche | Date d'achèvement de la recherche | Examinateur |
|---|---|---|
| LA HAYE | 20 janvier 2003 | Willekens, G |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03 82 (P04C02)

**EP 1 295 594 A1**

**ANNEXE AU RAPPORT DE RECHERCHE EUROPEENNE
RELATIF A LA DEMANDE DE BREVET EUROPEEN NO.**  EP 02 29 1970

La présente annexe indique les membres de la famille de brevets relatifs aux documents brevets cités dans le rapport de recherche européenne visé ci-dessus.
Lesdits members sont contenus au fichier informatique de l'Office européen des brevets à la date du
Les renseignements fournis sont donnés à titre indicatif et n'engagent pas la responsabilité de l'Office européen des brevets.

20-01-2003

| Document brevet cité au rapport de recherche | | | Date de publication | Membre(s) de la famille de brevet(s) | | | Date de publication |
|---|---|---|---|---|---|---|---|
| EP 968704 | A | | 05-01-2000 | FR | 2780644 | A1 | 07-01-2000 |
| | | | | BR | 9902771 | A | 02-05-2000 |
| | | | | DE | 69900038 | D1 | 11-01-2001 |
| | | | | DE | 69900038 | T2 | 20-09-2001 |
| | | | | EP | 0968704 | A1 | 05-01-2000 |
| | | | | ES | 2154503 | T3 | 01-04-2001 |
| | | | | JP | 3228916 | B2 | 12-11-2001 |
| | | | | JP | 2000026273 | A | 25-01-2000 |
| | | | | US | 2002187174 | A1 | 12-12-2002 |
| US 5952285 | A | | 14-09-1999 | AU | 642228 | B2 | 14-10-1993 |
| | | | | AU | 7422591 | A | 17-10-1991 |
| | | | | BR | 9101413 | A | 26-11-1991 |
| | | | | CA | 2040150 | A1 | 11-10-1991 |
| | | | | CN | 1057480 | A | 01-01-1992 |
| | | | | CS | 9101010 | A2 | 12-11-1991 |
| | | | | DE | 69122646 | D1 | 21-11-1996 |
| | | | | DE | 69122646 | T2 | 20-03-1997 |
| | | | | EG | 19920 | A | 31-10-1996 |
| | | | | EP | 0452106 | A2 | 16-10-1991 |
| | | | | ES | 2097183 | T3 | 01-04-1997 |
| | | | | FI | 911710 | A | 11-10-1991 |
| | | | | FR | 2661842 | A1 | 15-11-1991 |
| | | | | GB | 2245280 | A | 02-01-1992 |
| | | | | HK | 1004899 | A1 | 11-12-1998 |
| | | | | HU | 61327 | A2 | 28-12-1992 |
| | | | | IL | 97805 | A | 24-01-1995 |
| | | | | IT | 1245047 | B | 13-09-1994 |
| | | | | JP | 2080938 | C | 09-08-1996 |
| | | | | JP | 4227838 | A | 17-08-1992 |
| | | | | JP | 7063605 | B | 12-07-1995 |
| | | | | KR | 215229 | B1 | 16-08-1999 |
| | | | | NO | 911401 | A ,B, | 11-10-1991 |
| | | | | NZ | 237789 | A | 25-11-1994 |
| | | | | PL | 172315 | B1 | 30-09-1997 |
| | | | | SK | 281633 | B6 | 11-06-2001 |
| | | | | TR | 27723 | A | 22-06-1995 |
| | | | | ZA | 9102664 | A | 30-12-1992 |
| | | | | AT | 148158 | T | 15-02-1997 |
| | | | | AU | 665766 | B2 | 18-01-1996 |
| | | | | AU | 2133092 | A | 11-03-1993 |
| | | | | BG | 61079 | B1 | 31-10-1996 |
| | | | | BG | 96827 | A | 24-03-1994 |
| | | | | BR | 9203375 | A | 20-04-1993 |
| | | | | CA | 2077253 | A1 | 01-03-1993 |
| | | | | CN | 1073973 | A | 07-07-1993 |

EPO FORM P0460

Pour tout renseignement concernant cette annexe : voir Journal Officiel de l'Office européen des brevets, No.12/82

16

**ANNEXE AU RAPPORT DE RECHERCHE EUROPEENNE**
**RELATIF A LA DEMANDE DE BREVET EUROPEEN NO.**                    EP 02 29 1970

La présente annexe indique les membres de la famille de brevets relatifs aux documents brevets cités dans le rapport de recherche européenne visé ci-dessus.
Lesdits members sont contenus au fichier informatique de l'Office européen des brevets à la date du
Les renseignements fournis sont donnés à titre indicatif et n'engagent pas la responsabilité de l'Office européen des brevets.

20-01-2003

| Document brevet cité au rapport de recherche | | Date de publication | Membre(s) de la famille de brevet(s) | | Date de publication |
|---|---|---|---|---|---|
| US 5952285 | A | | CZ | 9202674 A3 | 12-05-1993 |
| | | | DE | 69216955 D1 | 06-03-1997 |
| | | | DE | 69216955 T2 | 12-06-1997 |
| | | | EG | 20081 A | 31-05-1997 |
| | | | EP | 0530708 A2 | 10-03-1993 |
| | | | ES | 2099775 T3 | 01-06-1997 |
| | | | FI | 923867 A | 01-03-1993 |
| | | | GB | 2259519 A ,B | 17-03-1993 |
| | | | HU | 63451 A2 | 30-08-1993 |
| | | | IL | 102950 A | 31-03-1996 |
| | | | JP | 3193143 B2 | 30-07-2001 |
| | | | JP | 5209198 A | 20-08-1993 |
| | | | KR | 266768 B1 | 15-09-2000 |
| | | | MX | 9205011 A1 | 30-06-1994 |
| EP 1184031 | A | 06-03-2002 | FR | 2813189 A1 | 01-03-2002 |
| | | | CN | 1342452 A | 03-04-2002 |
| | | | EP | 1184031 A2 | 06-03-2002 |
| | | | JP | 2002145736 A | 22-05-2002 |
| | | | US | 2002058010 A1 | 16-05-2002 |
| EP 1166747 | A | 02-01-2002 | FR | 2810542 A1 | 28-12-2001 |
| | | | BR | 0103382 A | 13-02-2002 |
| | | | CN | 1340340 A | 20-03-2002 |
| | | | EP | 1166747 A2 | 02-01-2002 |
| | | | JP | 2002097500 A | 02-04-2002 |

EPO FORM P0460

Pour tout renseignement concernant cette annexe : voir Journal Officiel de l'Office européen des brevets, No.12/82